(19) Europäisches Patentamt
European Patent Office
Office européen des brevets

(11) Veröffentlichungsnummer: **0 148 499**
**A2**

# (12) EUROPÄISCHE PATENTANMELDUNG

(21) Anmeldenummer: **84116307.4**

(22) Anmeldetag: **27.12.84**

(51) Int. Cl.4: **C 07 C 103/76**, C 07 F 7/18, A 01 N 37/36, A 01 N 55/00

(30) Priorität: **07.01.84 DE 3400401**

(43) Veröffentlichungstag der Anmeldung: **17.07.85 Patentblatt 85/29**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Salzburg, Herbert, Dr., Hahnenweg 3, D-5000 Köln 80 (DE)**
Erfinder: **Fauss, Rudolf, Dr., Gerstenkamp 10, D-5000 Köln 80 (DE)**
Erfinder: **Findeisen, Kurt, Dr., In der Follmühle 10, D-5068 Odenthal (DE)**
Erfinder: **Homeyer, Bernhard, Dr., Obere Strasse 28, D-5090 Leverkusen 3 (DE)**

(54) **Substituierte Malonsäurederivate, Verfahren zu ihrer Herstellung und ihre Verwendung als Schädlingsbekämpfungsmittel.**

(57) Die vorliegende Erfindung betrifft neue substituierte Malonsäurederivate der allgemeinen Formel I

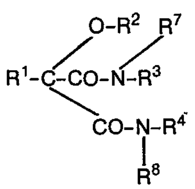

I

in welcher
$R^1$, $R^2$, $R^3$, $R^4$, $R^7$, $R^8$ die in der Beschreibung angegebene Bedeutung haben, sowie Verfahren zu ihrer Herstellung und ihre Verwendung als Schädlingsbekämpfungsmittel, insbesondere Insektizide.

EP 0 148 499 A2

BAYER AKTIENGESELLSCHAFT 5090 Leverkusen, Bayerwerk
Konzernverwaltung RP
Patentabteilung

Rt/bo-c

Ia

Substituierte Malonsäurederivate, Verfahren zu ihrer Herstellung und ihre Verwendung als Schädlingsbekämpfungsmittel

Die vorliegende Erfindung betrifft neue substituierte Malonsäurederivate, Verfahren zu ihrer Herstellung und ihre Verwendung als Schädlingsbekämpfungsmittel.

Es ist bereits bekannt geworden, daß Malonsäurederivate wie 3,4-Dichlorphenyl-malonsäurediamid insektizide Wirksamkeit besitzen (DE-OS 3 140 275). Ihre Wirkung ist bei niedrigen Aufwandkonzentrationen nicht immer befriedigend.

Es wurde gefunden, daß die substituierten Malonsäurederivate der allgemeinen Formel I sich hervorragend zur Schädlingsbekämpfung eignen:

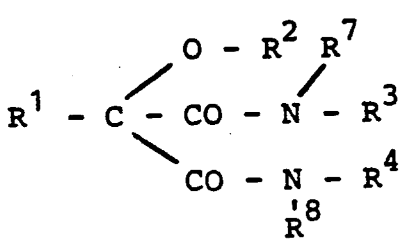

I

in welcher

$R^1$ für Aryl oder Heteroaryl, die gegebenenfalls substituiert sein können, steht

$R^2$ für Wasserstoff, Trialkylsilyl sowie für Alkyl, Cycloalkyl, Alkenyl, Alkinyl, Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$- CO - NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können stehen,

$R^3$ steht für Wasserstoff oder den Rest $R^4$

$R^7$ und $R^8$ unabhängig voneinander für Wasserstoff, Alkyl oder Aryl stehen und

$R^4$ für einen Rest der Formel

$$-CH_2-R^9$$

steht, wobei

$R^9$ für Hydroxyl, Alkoxy, Aryloxy, die gegebenenfalls substituiert sein können, Alkylamino, Cycloalkylamino, Arylamino, Dialkylamino, Alkenylamino, Diarylamino, Aralkylamino oder über N gebundene stickstoffhaltige gesättigte heterocyclische Reste, die gegebenenfalls weitere Heteroatome enthalten.

Es wurde ferner gefunden, daß man die Verbindungen der Formel I

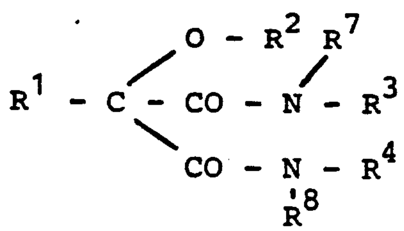

I

wobei

$R^1$ für Aryl oder Heteroaryl, die gegebenenfalls substituiert sein können, steht

$R^2$ für Wasserstoff, Trialkylsilyl sowie für Alkyl, Cyloalkyl, Alkenyl, Alkinyl, Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$-CO-NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können stehen,

$R^3$ steht für Wasserstoff oder den Rest $R^4$,

$R^7$ und $R^8$ für Wasserstoff, Alkyl oder Aryl stehen und

$R^4$ für einen Rest der Formel

$$-CH_2-R^9$$

steht, wobei

$R^9$ für Hydroxy, Alkoxy, Aryloxy, die gegebenenfalls substituiert sein können, Alkylamino, Arylamino, Aralkylamino, Dialkylamino, Diarylamino, Cycloalkylamino, Alkenylamino, über N gebundene stickstoffhaltige gesättigte heterocyclische Reste, die gegebenenfalls weitere Heteroatome enthalten,

erhält, indem man Verbindungen der Formel II

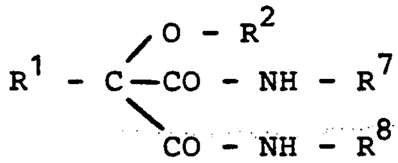

II

wobei

$R^1$, $R^2$, $R^7$ und $R^8$ die oben angegebene Bedeutung haben,

mit Formaldehyd oder Formaldehyd-abspaltenden Verbindungen in Gegenwart einer Säure oder Base umsetzt und gegebenenfalls gleichzeitig oder anschließend mit Alkylaminen, Cycloalkylaminen, Alkenylaminen, Arylaminen, Dialkylaminen, Diarylaminen, Aralkylaminen oder N-haltigen gesättigten Heterocyclen, die gegebenenfalls weitere Heteroatome enthalten können, oder mit aliphatischen oder aromatischen OH-Verbindungen umsetzt.

Die erfindungsgemäßen substituierten Malonsäurederivate sind besonders geeignet zur Bekämpfung von Insekten und Spinnenmilben. Sie zeichnen sich dabei durch eine erheblich bessere Wirkung aus als die aus dem Stand der Technik für diese Indikationen bekannten Verbindungen. Sie besitzen außerdem günstige Werte der Warmblütertoxizität.

Bevorzugt sind die neuen substituierten Malonsäurederivate der allgemeinen Formel I

in welcher

$R^1$ für Phenyl steht, das gegebenenfalls gleich oder verschieden durch einen oder mehrere der

folgenden Reste substituiert sein kann: Halogen, insbesondere Chlor, Brom, Fluor, Nitro, Amino, OH, CN, $C_{1-4}$-Alkyl insbesondere Methyl, $C_{1-4}$-Halogenalkyl insbesondere Trifluormethyl, Trichlormethyl, Pentafluorethyl, $C_{1-4}$-Alkoxy, Methylendioxy, Ethylendioxy, $C_{1-4}$-Halogenalkoxy, insbesondere Trifluormethoxy, Pentafluorethoxy, Difluormethylendioxy, halogensubstituiertes Ethylendioxy, $C_{1-4}$-Alkylthio, $C_{1-4}$-Halogenalkylthio insbesondere Trifluormethylthio, $C_{2-8}$-Alkoxyalkyl, $C_{2-8}$-Halogenalkoxyalkyl, $C_{1-4}$-Alkylsulfonyl, insbesondere Methylsulfonyl, $C_{1-4}$-Halogenalkylsulfonyl, Carboxyl, Carbalkoxy, insbesondere Methoxycarbonyl, sowie für den Rest $C_{1-4}$-Alkoxy-N=CH-, insbesondere $CH_3$-O-N=CH-, ferner für Phenyl, Phenyloxy, Thiophenyl die gegebenenfalls durch Halogen oder $C_{1-4}$-Alkyl substituiert sein können, sowie für Carboxylalkoxy mit 2 - 4 C-Atomen wie Carboxymethoxy,

$R^1$ steht, ferner bevorzugt für Heteroaryl wie Pyridinyl, Pyrimidinyl, Triazinyl, Isoxazolyl, Thiazolyl, Oxadiazolyl, Imidazolyl, Triazolyl, Furanyl, Thiophenyl, die gegebenenfalls ein oder mehrfach gleich oder verschieden durch Halogen insbesondere Chlor, $C_{1-4}$-Alkyl, insbesondere Methyl, Ethyl, $C_{1-4}$-Alkoxy, insbesondere Methoxy, Ethoxy substituiert sein können.

$R^1$ steht, außerdem bevorzugt für 3-Nitrophenyl, 3-Jodphenyl, Biphenyl, 4-Trimethylsilyloxyphenyl, 4-Chlor-3-nitrophenyl, 3-Chlor-4-nitrophenyl, 3,4,5-Trichlorphenyl, 3,5-Dichlor-4-fluorphenyl, 4-Difluormethylphenyl, 3-Nitro-4-fluorphenyl, 3-Fluor-4-nitrophenyl, 3-Trifluormethyl-4-chlorphenyl, 4-Trifluormethyl-3-chlorphenyl, 3-Chlor-5-trifluormethylphenyl, 3,4-Di(trifluormethyl)-phenyl, 3-Trifluormethyl-4,5-dichlorphenyl, 4-Trifluormethyl-3,5-dichlorphenyl, 4-Trifluormethoxy-3-nitrophenyl, 4-Trifluormethoxy-3-bromphenyl, 4-Nitro-3-trifluormethoxyphenyl, 4-Brom-3-trifluormethoxyphenyl, 3-Nitro-4-trifluormethoxy-5-chlorphenyl, 4-Methoxy-3,5-dichlorphenyl, 4-Methyl-3,5-dichlorphenyl, 4-Fluor-3-bromphenyl, 4-Brom-3-fluorphenyl, 4-Chlor-3-methylphenyl, 4-Trifluormethylmercaptophenyl, 4-Trifluormethoxy-3-chlorphenyl, 3-Trifluormethyl-4-chlorphenyl, 4-Chlor-difluormethoxy-3-chlorphenyl, 4-Fluor-3-chlorphenyl, Pentafluorphenyl, 4-Fluor-3,5-dibromphenyl, 4-Fluor-3-chlor-5-bromphenyl, 4-Chlor-3,5-dibromphenyl, 4-Brom-3,5-dichlorphenyl, 3-Brom-4,5-dichlorphenyl, 3,4,5-Trifluorphenyl, 3,4,5-Tribromphenyl, 4-Amino-3,5-dichlorphenyl, 4-Hydroxy-3,5-dichlorphenyl.

Bevorzugt sind Verbindungen der Formel I, in welcher

$R^2$ für Wasserstoff, Trialkylsilyl mit 1 - 4 C-Atomen im Alkylteil, $C_{1-4}$-Alkyl, $C_{1-4}$-Alkylcarbonyl, Benzoyl, das gegebenenfalls gleich oder verschieden durch einen oder mehrere der folgenden Reste (A) substituiert sein kann. (A) steht für Halogen, insbesondere Chlor, Brom, Fluor, Nitro, Amino, CN, $C_{1-4}$-Alkyl, insbesondere Methyl, $C_{1-4}$-Halogenalkyl, insbesondere Trifluormethyl, Pentafluorethyl, $C_{1-4}$-Alkoxy, $C_{1-4}$-Halogenalkoxy, insbesondere Trifluormethoxy, Pentafluorethoxy, Methylendioxy, Ethylendioxy, Difluormethylendioxy, halogensubstituierte Ethylendioxy wie Trifluorethylendioxy, $C_{1-4}$-Alkylthio, $C_{1-4}$-Halogenalkylthio insbesondere Trifluormethylthio, $C_{2-8}$-Alkoxyalkyl, $C_{2-8}$-Halogenalkoxyalkyl, $C_{1-5}$-Alkylsulfenyl, Phenylsulfenyl, die gegebenenfalls substituiert sein können, $C_{1-4}$-Alkylsulfonyl, insbesondere Methylsulfonyl, $C_{1-4}$-Halogenalkylsulfonyl, Carbalkoxy, insbesondere Methoxycarbonyl, sowie für den Rest $C_{1-4}$-Alkoxy-N=CH-, insbesondere $CH_3$-O-N=CH-, ferner für Phenyl, Phenyloxy, Thiophenyl die gegebenenfalls durch Halogen oder $C_{1-4}$-Alkyl substituiert sein können, sowie für Carboxyalkoxy mit 2 - 4 C-Atomen, wie Carboxymethoxy;

$R^2$ steht ferner bevorzugt für $C_{1-4}$-Alkoxycarbonyl, Phenoxycarbonyl, das gege-

benenfalls einen oder mehrere der Reste (A) substituiert sein kann, $C_{1-4}$-Alkylsulfonyl, Phenylsulfonyl, das gegebenenfalls durch einen oder mehrere der Reste (A) substituiert sein kann, $C_{1-4}$-Alkylaminosulfonyl, $C_{1-5}$-Alkylsulfenyl, Phenylsulfenyl, die gegebenenfalls substituiert sein können, Di-$C_{1-4}$-alkylaminosulfonyl, Phenylaminosulfonyl, das gegebenenfalls durch einen oder mehrere der Reste (A) substituiert sein kann, Phenyl-$C_{1-4}$-alkyl-aminosulfonyl.

Bevorzugt sind Verbindungen der Formel I,

in welcher

$R^3$ für Wasserstoff oder den Rest $R^4$ steht.

$R^7$ und $R^8$ stehen bevorzugt für Wasserstoff, $C_{1-4}$-Alkyl, Phenyl, das gegebenenfalls durch Halogen substituiert ist. Ganz besonders bevorzugt ist Wasserstoff und Methyl.

$R^4$ steht bevorzugt für

$$-CH_2-R^9$$

wobei

$R^9$ für Hydroxyl, $C_{1-4}$-Alkoxy, Phenoxy, das gegebenenfalls durch einen oder mehrere

der Reste (A) substituiert sein kann, $C_{1-8}$-Alkylamino, $C_{5-6}$-Cycloalkylamino, Phenylamino, das gegebenenfalls durch einen oder mehrere der Reste (A) substituiert ist, Di-$C_{1-8}$-alkylamino, Phenyl-$C_{1-4}$-alkylamino, gesättigte, über N gebundenen stickstoffhaltige Heterocyclen mit 5 - 6 Ringatomen wie Morpholin, Pyrimidin, Piperidin steht.

Besonders bevorzugt sind Verbindungen der Formel I

in welcher

$R^1$ für Phenyl, das gegebenenfalls durch Halogen, insbesondere Fluor oder Chlor, $C_{1-4}$-Alkyl, insbesondere Methyl, $C_{1-4}$-Alkoxy, $C_{1-4}$-Halogenalkoxy, $C_{1-4}$-Halogenalkyl, $NH_2$, $CH_3O-N=CH-$, oder Nitro substituiert ist, steht.

Ganz besonders zu erwähnen sind Verbindungen der allgemeinen Formel I, in welcher

$R^1$ für Phenyl steht, das gegebenenfalls ein bis dreifach gleich oder verschieden in 3-, 4- oder 5-Stellung durch Halogen insbesondere Fluor, Chlor, Brom, Jod substituiert ist,

$R^2$ für $C_{1-4}$-Alkyl, insbesondere Methyl, gegebenenfalls halogensubstituiertes $C_{1-4}$-Alkylaminosulfonyl, $C_{1-4}$-Alkylcarbonyl, insbesondere

Methylcarbonyl, gegebenenfalls durch Halogen substituiertes Phenylcarbonyl, Phenylsulfenyl, $C_{1-4}$-Alkylsulfenyl, Trialkylsilyl,insbesondere Trimethylsilyl sowie für

$-CO-NR^5R^6$, wobei $R^5$ für Wasserstoff steht und $R^6$ für gegebenenfalls durch Halogen substituiertes Phenyl oder Phenylcarbonyl oder für $C_{1-4}$-Alkyl oder $C_{1-4}$-Alkylcarbonyl steht,

$R^4$ für $-CH_2-R^9$ steht, wobei

$R^9$ für $C_{1-4}$-Alkylamino, Phenylamino, $C_{2-8}$-Dialkylamino, Morpholino, Piperidino steht.

Besonders seien genannt Verbindungen der Formel I,

in welcher

$R^1$ für Phenyl steht, das gegebenenfalls ein oder mehrfach durch Chlor substituiert ist und

$R^2$ für Wasserstoff oder Trimethylsilyl steht,

$R^3$ für Wasserstoff oder $R^4$ steht, und

$R^4$ für Methylamino, Ethylamino, Morpholino oder Piperidino steht.

Im Einzelnen seien die Verbindungen der folgenden Tabelle genannt:

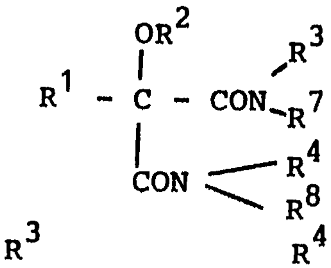 I

| $R^1$ | $R^2$ | $R^3$ | $R^4$ | $R^7$ | $R^8$ |
|---|---|---|---|---|---|
| 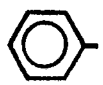 | $-H$ | $-H$ | $-CH_2OCH_3$ | $-H$ | $-H$ |
| 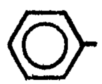 | $-H$ | $-CH_2OCH_3$ | $-CH_2OCH_3$ | $-H$ | $-H$ |
| 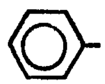 | $-CH_3$ | $-H$ | $-CH_2OH$ | $-H$ | $-H$ |
| 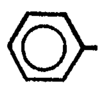 | $-H$ | $-CH_3$ | $-CH_2NH-C_6H_5$ | $-CH_3$ | $-CH_2NH-C_6H_5$ |
| 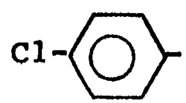 | $-H$ | $-H$ | $-CH_2OC_2H_5$ | $-H$ | $-H$ |
| 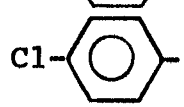 | $-H$ | $-CH_2OCH_3$ | $-CH_2OCH_3$ | $-H$ | $-H$ |
| 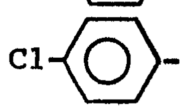 | $-CH_3$ | $-CH_2OH$ | $-CH_2OH$ | $-CH_3$ | $-CH_3$ |
| 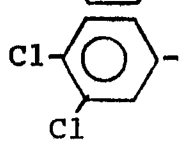 | $-H$ | $-CH_2OC_2H_5$ | $-CH_2OC_2H_5$ | $-H$ | $-H$ |

| $R^1$ | $R^2$ | $R^3$ | $R^4$ | $R^7$ | $R^8$ |
|---|---|---|---|---|---|
| 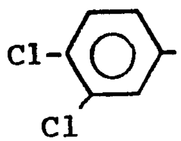 | -H | $-CH_2N(CH_3)_2$ | $-CH_2N(CH_3)_2$ | $-CH_3$ | $-CH_3$ |
| 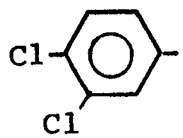 | $-CH_3$ | $-CH_2N$ 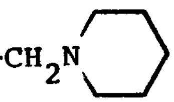 | $-CH_2-N$ 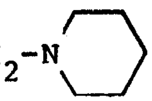 | -H | -H |
| 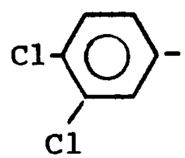 | $-SI(CH_3)_3$ | $-CH_2N$ 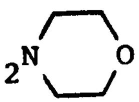 | $-CH_2-N$ 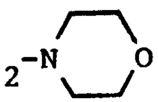 | -H | -H |

Die Umsetzung der Verbindungen der Formel II mit Formaldehyd erfolgt gegebenenfalls in Gegenwart von Säureakzeptoren, sowie gegebenenfalls in Gegenwart eines Verdünnungsmittels. Der Reaktionsablauf kann z.B. durch das folgende Formelschema wiedergegeben werden:

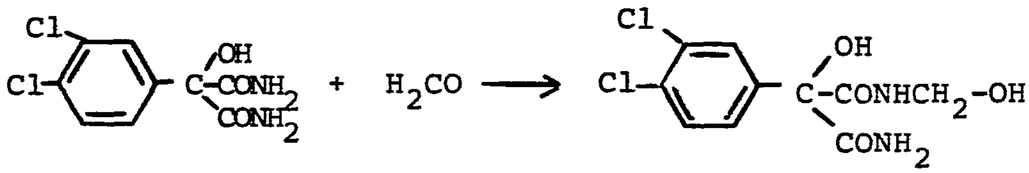

Formaldehyd wird äquimolar bis etwa 10-fach im Überschuß eingesetzt.

Als Verbindungen der Formeln II werden bevorzugt diejenigen eingesetzt, die in den Substituenten $R^3$, $R^4$ die bei den Verbindungen der Formel I genannten bevorzugten Bedeutungen besitzen. Die Verbindungen der Formel II sind bekannt (Deutsche Offenlegungsschrift 31 40 275). Sie lassen sich nach den dort angegebenen Verfahren herstellen. Im einzelnen seien die folgenden Verbindungen der Formel II genannt:

Phenyl-hydroxy-malonsäurediamid, 2-, 3-, 4-Chlorphenylhydroxy-malonsäurediamid, 2,3-Dichlorphenyl-hydroxy-malonsäurediamid, 3,4-Dichlorphenyl-hydroxy-malonsäurediamid, 3,5-Dichlorophenyl-hydroxy-malonsäurediamid, 2,4-Dichlorophenyl-hydroxy-malonsäurediamid, 2,5-Dichlorophenyl-hydroxy-malonsäurediamid, 2,6-Dichlorophenyl-hydroxy-malonsäurediamid, 2-, 3-, 4-Nitrophenylhydroxy-malonsäurediamid, 2-Chloromethylphenyl-hydroxy-

malonsäurediamid, 2-, 3-, 4-Trifluormethylphenyl-hydroxymalonsäurediamid, 2-, 3-, 4-Methoxyphenyl-hydroxy-malonsäurediamid, 2,6-Dimethoxyphenyl-hydroxy-malonsäurediamid, 2-, 3-, 4-Tolyl-hydroxy-malonsäurediamid, 2-, 3-, 4-Trifluormethoxyphenyl-hydroxy-malonsäurediamid, 2-, 3-, und 4-Fluorphenyl-hydroxymalonsäurediamid, 3,4-Dichlorphenyl-hydroxy-malonsäureamid-methylamid, Phenylhydroxy-malonsäure-diethylamid-amid, 3,5-Dichlorphenylhydroxy-malonsäureamid-morpholinylamid, 3,4-Dichlorphenyl-hydroxy-malonsäure-bis-isopentylamid, Phenylhydroxy-malonsäure-bismethylamid, Cyclohexyl-hydroxy-malonsäurediamid, 2,3,4-; 2,3,6-; 3,4,5-Trichlorphenyl-hydroxy-malonsäure-diamid, 2,3,4,5-; 2,3,5,6-Tetrachlorphenyl-hydroxy-malonsäurediamid, Pentachlorphenylhydroxy-malonsäurediamid.

Als Formaldehyd abspaltende Verbindungen seien genannt polymere Formaldehyde wie Metaldehyd, Paraldehyd sowie Formaldehydacetale wie Formaldehyddiethylacetal.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethyleter, Tetrahydrofuran und Dioxan, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen können alle üblichen Säurebindemittel verwendet werden. Hierzu gehören vorzugsweise Alkalicarbonate, -hydroxide oder -alkoholate, wie Natrium- oder Kaliumcarbonat, Natrium- und Kaliumhydroxid, Natrium- und Kaliummethylat bzw. -ethylat, ferner aliphatische, aromatische oder heterocyclische Amine, beispielsweise Trimethylamin, Triethylamin, Tributylamin, Dimethylanilin, Dimethylbenzylamin und Pyridin.

Als Säuren kommen Lewis-Säuren in Betracht, wie Bortrifluorid, Aluminiumtrichlorid, Titantetrachlorid sowie Mineralsäuren wie Schwefelsäure, Halogenwasserstoffe, Phosphorsäure.

Die Reaktionstemperatur wird zwischen etwa 0°C und 130°C, vorzugsweise zwischen etwa 20°C und 60°C gehalten. Das Verfahren wird vorzugsweise bei Normaldruck durchgeführt.

Nach Beendigung der Reaktion kann das Reaktionsgemisch in üblicher Weise aufbereitet werden, um die Verbindungen der Formel I, in welcher $R^9$ für OH steht, zu isolieren. Das Reaktionsgemisch kann aber auch sofort zur Weiterreaktion mit Aminen eingesetzt werden.

Dazu wird das Reaktionsgemisch in einem der genannten Lösungsmittel, bevorzugt in Ethern wie Dioxan oder Dialkylether oder in Ketonen wie Aceton mit einem mindestens äquimolaren Anteil der Aminkomponenten versetzt, wobei saure und basische Katalysatoren in der üblichen Weise zum Einsatz kommen. Die Reaktion wird zwischen 0°C und 130°C, bevorzugt 40°C - 80°C bis zum Stillstand geführt, was normalerweise nach 1 bis 6 Stunden der Fall ist.

Als Katalysatoren seien die bei der Durchführung der Umsetzung mit Formaldehyd genannten sauren und basischen Katalysatoren genannt. Bevorzugt sind die sauren Katalysatoren.

Als Amine die eingesetzt werden, seien genannt: Methylamin, Ethylamin, Propylamin, Isopropylamin, Butylamin, iso-Butylamin, tert.-Butylamin, Hexylamin, Dodecylamin, 2-Ethylhexylamin, Tetradecylamin, Hexadecylamin, Octadecylamin, Allylamin, 2-Methoxyethylamin, 2-Ethoxypropylamin, 3-Butoxypropylamin, 3-Aminopropansäure-2-methylpropylester, 6-Aminohexanitril, Lysinester, Aminoundecansäureester, Cyclohexylamin, Trimethylcyclohexylamin, 2-Borbornylmethylamin, Anilin, o,m,p-Chloranilin, 2,3-, 2,4-, 2,5-, 2,6-Dichloranilin, 3,4-, 3,5-Dichloranilin, p-o-Nitroanilin, m,o,p-Tolylamin, 3-Trifluormethylanilin, 3-Chlor-4-methylanilin, 4-Chlor-3-methylanilin, Benzylamin, Phenylcyclohexylamin, Naphthylamin, wie auch sek. Amine, z.B. Dimethylamin, Diethylamin, Morpholin, Piperidin, Piperazin, Pyrazol, Imidazol.

Nach Beendigung der Reaktion werden die Verbindungen der Formel I in üblicher Weise isoliert.

Nach einer Variante des Verfahrens können Verbindungen der Formel I, in denen der Rest $R^8$ eine andere Bedeutung als Hydroxy hat, hergestellt werden. Dazu werden die Verbindungen der Formel II mit Formaldehyd und den Aminen in einer einstufigen Synthese zur Reaktion gebracht. Die Reaktion wird dabei so durchgeführt, daß man die Komponenten in einem mindestens äqumolaren Verhältnis, bevorzugt mit der 3- bis 6-fachen Menge Formaldehyd, bezogen auf die Verbindungen der Formel II und einem Überschuß an Amin in der gleichen Menge in einem der genannten Lösungsmittel, besonders bevorzugt in Dioxan, Diethylether, Tetrahydrofuran, Aceton mit dem Katalysator, z. B. Kaliumcarbonat, aber auch Bortrifluorid-Etherat bei Temperaturen zwischen 30°C und 100°C rührt.

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii,

Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variiert werden. Die Wirkstoffkonzentration

der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten, vorzugsweise von ektoparasitierenden Insekten auf dem Gebiet der Tierhaltung und der Tierzucht.

Die Anwendung der erfindungsgemäßen Wirktoffe geschieht hier in bekannter Weise, wie durch orale Anwendung, durch dermale Anwendung in Form beispielsweise des Tauchens (Dippen), Sprühens (Sprayen), Aufgießens (pour-on and spot-on) und des Einpuderns.

Herstellungsbeispiele

Herstellung der Ausgangssubstanzen

Beispiel a

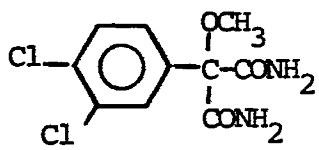

30 g (0,114 Mol) 3,4-Dichlor-phenyl-hydroxy-malonsäurediamid werden in 60 ml Dimethylsulfoxid vorgelegt. Dann werden 6,38 g (0,144 Mol) Kaliumhydroxid in 150 ml Wasser, anschließend 16,1 g (0,114 Mol) Methyliodid zugetropft. Die Reaktion ist exotherm: Die Temperatur steigt auf etwa 50°C. Man läßt abkühlen und rührt 16 Stunden bei 20°C nach. Der ausgefallene Feststoff wird abgesaugt und mit Wasser und dann mit Petrolether gewaschen.

Nach Umkristallisieren aus Essigester erhält man 14,1 g (44,3 % der Theorie) 2-(3,4-Dichlorphenyl)-2-methoxy-malonsäurediamid vom Schmelzpunkt 211 bis 213°C.

Beispiel b

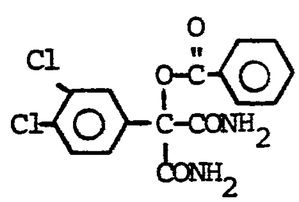

13,1 g 3,4-Dichlorphenylhydroxymalonsäurediamid wurden in 200 ml Benzoylchlorid 9 Stunden auf 110°C erhitzt.

Anschließend wurde auf 5°C abgekühlt, abgesaugt, der Niederschlag mit Toluol gewaschen und anscließend aus 550 ml i-Propanol umkristallisiert. Es wurden 10 g 3,3-Dichlorphenylbenzoyloxymalonsäurediamid isoliert: Fp. 225°C unter Zersetzung.

Beispiel c

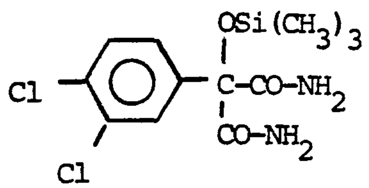

3 g (0,0114 Mol) 3,4-Dichlor-phenyl-hydroxy-malonsäurediamid, 1,12 g (0,0114 Mol) Trimethylsilylcyanid und 0,9 g (0,0114 Mol) Pyridin werden 6 Stunden auf 100°C (Badtemperatur) erhitzt. Nach Entfernen des Pyridins im Wasserstrahlvakuum wird der Rückstand aus Petrolether umkristallisiert.

Man erhält 3,45 g (90,3 % der Theorie) 2-(3,4-Dichlorphenyl)-2-trimethylsilyloxy-malonsäurediamid vom Schmelzpunkt 156°C.

Beispiel 1

78 g (0,3 Mol) 3,4-Dichlorphenyl-hydroxymalonsäurediamid und 27 g Paraformaldehyd (0,9 Mol) werden in 1 Liter Aceton mit 0,5 g Kaliumcarbonat 15 Min. am Rückfluß gerührt und anschließend heiß abfiltriert. Beim Abkühlen fällt N,N'-Bishydroxymethylen-3,4-dichlorphenyl-hydroxymalonsäurediamid aus. Fp. 118 - 120°C, Ausbeute 43 g (49 % d.Th.). Die Mutterlauge enthält vorwiegend das Monohydroxymethylen-Produkt.

Beispiel 2

1:1-Gemisch von Mono- und Bishydroxymethylenprodukt:
78 g (0,3 Mol) des Ausgangsproduktes wie in Beispiel 1 werden mit 27 g Paraformaldehyd und 0,5 g Kaliumoxid in 1 Liter Aceton 15 Min. gekocht, heiß filtriert und anschließend eingeengt. Es bleibt ein farbloses Öl, das ein 1:1-Gemisch aus Mono- und Bishydroxymethylen-Produkt von 3,4-Dichlorphenyl-hydroxymalonsäurediamid darstellt.

Beispiel 3

Bis-piperidinomethylen-Produkt:
5,2 g (0,02 Mol) 3,4-Dichlorphenyl-hydroxymalonsäurediamid und 4 g 37 %ige wäßrige Formaldehydlösung werden mit 3 g Piperidin und 3 Tropfen Bortrifluorid-Etherat in 50 ml Dioxan bei 50°C gerührt. Nach ca. 7 Stunden wird die Reaktion mit 0,5 ml Triethylamin unterbrochen und das Reaktionsgemisch eingeengt. Der feste Rückstand wird mit Ether aufgenommen, verrührt, abgesaugt und mit

Ether nachgewaschen. Ausbeute: 6,0 g Bis-piperidinomethylen-3,4-dichlorphenyl-hydroxymalonsäureamid (66 % d.Th.). Aus der Mutterlauge ist weiteres Produkt isolierbar. Fp.: 159 - 160°C.

Beispiel 4

Analog Beispiel 3 werden im gleich großen Ansatz mit 5,8 g 50 %iger wäßriger Dimethylamin-Lösung 2,5 g Bis-dimethylaminomethylen-3,4-dichlorphenyl-hydroxymalonsäurediamid (33 % d.Th.) erhalten. Fp.: 98 - 100°C. Mit gasförmigem Dimethylamin werden 5,8 g Produkt erhalten (77 % d.Th.).

Beispiel 5

3,21 g Bishydroxymethylen-3,4-dichlorphenyl-hydroxymalonsäurediamid (0,01 Mol) werden mit 3 g Piperidin und 3 Tropfen Bortrifluorid in 50 ml Dioxan 8 Stunden auf 50°C gehalten. Nach Zugabe von 0,5 ml Triethylamin wird eingeengt, mit Ether aufgenommen und abgesaugt. Man erhält 6,7 g Bis-piperidinomethylen-3,4-dichlorphenyl-hydroxymalonsäurediamid (73 % d.Th.) vom Fp.: 195°C.

Beispiel 6

5,2 g Ausgangsverbindung aus Beispiel 1 werden mit 3,2 g 37%iger wäßriger Formalinlösung und 2 g Morpholin in 20 ml Dioxan mit 2 ml konz. Schwefelsäure 6 Stunden bei 40°C

gerührt. Nach Zugabe von 4 ml Triethylamin wird im Vakuum eingeengt und der Rückstand mit 30 ml Diethylether aufgenommen. Der ausgefallene Feststoff ist ein 1:1-Gemisch aus Mono- und Bismorpholinomethylen-3,4-dichlorphenyl-hydroxymalonsäurediamid. Ausbeute: 3,7 g.

Beispiel A

Grenzkonzentrations-Test / Wurzelsystemische Wirkung

Testinsekt: Phaedon cochleariae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 6.

0148499



## T a b e l l e

Wurzelsystemische Wirkung
Phaedon cochleariae-Larven

| Wirkstoff (Konstitution) | Abtötungsgrad in % bei Wirkstoffkonzentration in ppm |
|---|---|
| 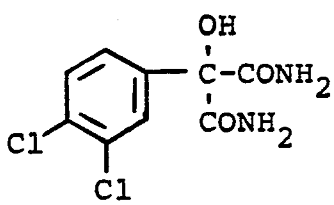 bekannt | 2,5 ppm = 0 % |
| 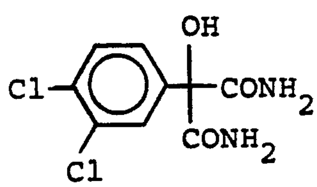 + 2 $CH_2O$ erfindungsgemäß | 2,5 ppm = 95 % |
| 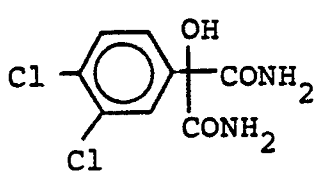 + 1 $CH_2O$ | 2,5 ppm = 100 % |

Beispiel B

Grenzkonzentrations-Test / Bodeninsekten

Testinsekt: Phorbia antiqua-Maden im Boden
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Wirkstoffe, Aufwandmengen und Resultate gehen aus der nachfolgenden Tabelle hervor:

## T a b e l l e

Bodeninsektizide
Phorbia antiqua-Maden im Boden

| Wirkstoff (Konstitution) | Abtötungsgrad in % bei Wirkstoffkonzentration in ppm |
|---|---|
| 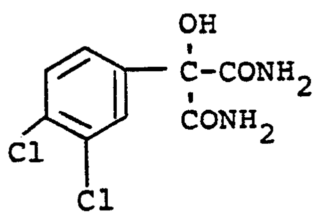 bekannt | 20 ppm = 0 % |
| 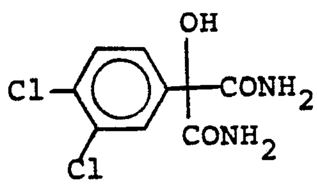 + 2 $CH_2O$ erfindungsgemäß | 20 ppm = 95 % |

Patentansprüche

1. Substituierten Malonsäurederivate der allgemeinen Formel I

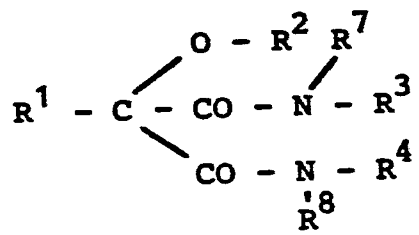

I

wobei

$R^1$ für Aryl oder Heteroaryl, die gegebenenfalls substituiert sein können, steht,

$R^2$ für Wasserstoff, Trialkylsilyl sowie für Alkyl, Cycloalkyl, Alkenyl, Alkinyl, Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl,Alkylaminosulfonyl, Dialikylaminosulfonyl, Arylaminosulfonyl, Arylalkylaminosulfonyl, die gegebenenfalls substituiert sein können, sowie für Reste der Formel

$$-CO-NR^5R^6$$

wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können, steht.

$R^3$ steht für Wasserstoff oder den Rest $R^4$,

$R^7$ und $R^8$ stehen unabhängig voneinander für Wasserstoff, Alkyl oder Aryl,

$R^4$ steht für einen Rest der Formel

$-CH_2-R^9$

$R^9$ steht für Hydroxyl, Alkoxy, Aryloxy, die gegebenenfalls substituiert sind, sowie für Alkylamino, Arylamino, Aralkylamino, Dialkylamino, Diarylamino, Cycloalkylamino, Alkenylamino, über N gebundene stickstoffhaltige gesättigte heterocyclische Reste, die gegebenenfalls weitere Heteroatome enthalten.

2. Verfahren zur Herstellung der Verbindungen der Formel I

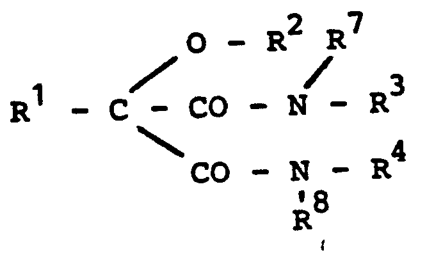

I

wobei

$R^1$ für Aryl oder Heteroaryl, die gegebenenfalls substituiert sein können, steht,

$R^2$ für Wasserstoff, Trialkylsilyl sowie für Alkyl, Cycloalkyl, Alkenyl, Alkinyl,

Aralkyl, Alkylcarbonyl, Arylcarbonyl, Alkoxycarbonyl, Aryloxycarbonyl, Alkylsulfenyl, Arylsulfenyl, Alkylsulfonyl, Arylsulfonyl, Alkylaminosulfonyl, Dialkylaminosulfonyl, Arylaminosulfonyl, Arylalkylaminosulfonyl, die gegebenenfalls substituiert sein können, sowie für Reste der Formel

$$-CO-NR^5R^6$$

steht, wobei

$R^5$ und $R^6$ unabhängig voneinander für Wasserstoff, Alkyl, Cycloalkyl, Aryl, Alkylaminocarbonyl, Arylaminocarbonyl, Alkylcarbonyl, Arylcarbonyl, Alkoxycarbonyl, Aryloxycarbonyl, Alkylsulfonyl, Arylsulfonyl, wobei diese Reste gegebenenfalls substituiert sein können steht,

$R^3$ für Wasserstoff oder den Rest $R^4$ steht,

$R^4$ für einen Rest der Formel

$$-CH_2-R^9$$

steht, wobei

$R^9$ für Hydroxyl, Alkoxy, Aryloxy die gegebenenfalls substituiert sind,

Alkylamino, Arylamino, Aralkylamino, Dialkylamino, Diarylamino, Cycloalkylamino, Alkenylamino, über N-gebundene stickstoffhaltige gesättigte heterocyclische Reste die gegebenenfalls weitere Heteroatome enthalten,

$R^7$ und $R^8$ unabhängig voneinander für Wasserstoff, Alkyl oder Aryl stehen,

dadurch gekennzeichnet, daß man Verbindungen der Formel II

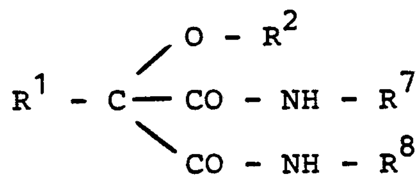

II

wobei

$R^1$, $R^2$, $R^7$ und $R^8$ die oben angegebene Bedeutung haben, mit Formaldehyd oder Formaldehyd abspaltenden Verbindungen in Gegenwart einer Base oder Säure umsetzt und gegebenenfalls gleichzeitig oder anschließend mit Alkylamin, Cycloalkylaminen, Alkenylaminen, Arylaminen, Dialkylaminen, Diarylaminen, Aralkylaminen oder N-haltigen gesättigten Heterocyclen die gegebenenfalls weitere Heteroatome enthalten können oder mit aliphatischen oder aromatischen OH-Verbindungen umsetzt.

3. Schädlingsbekämpfungsmittel, gekennzeichnet durch einen Gehalt an substituierten Malonsäurederivaten der allgemeinen Formel I gemäß Anspruch 1.

4. Verwendung von substituierten Malonsäurederivaten der Formel (I) gemäß Anspruch 1 zur Bekämpfung von Schädlingen.

5. Verfahren zur Bekämpfung von Schädlingen, dadurch gekennzeichnet, daß man substituierte Malonsäurederivate der Formel (I) gemäß Anspruch 1 auf Schädlinge und/oder ihren Lebensraum einwirken läßt.

6. Verfahren zur Herstellung von Schädlingsbekämpfungsmitteln, dadurch gekennzeichnet, daß man substituierte Malonsäurederivate der Formel (I) gemäß Anspruch 1 mit Streckmitteln und/oder oberflächenaktiven Mitteln vermischt.